Europäisches Patentamt

European Patent Office

Office européen des brevets

(11) Publication number: **0 355 411**
**A1**

## EUROPEAN PATENT APPLICATION

(21) Application number: 89113359.7

(22) Date of filing: 20.07.89

(51) Int. Cl.⁴: **A61B 17/58**

(30) Priority: 10.08.88 US 230563

(43) Date of publication of application:
28.02.90 Bulletin 90/09

(84) Designated Contracting States:
DE ES FR GB

(71) Applicant: ACE ORTHOPEDIC
MANUFACTURING
14105 South Avalon Blvd.
Los Angeles, CA 90061(US)

(72) Inventor: Zindrick, Michael R.
151 Juliet Ct.
Clarendon Hills Illinois 60514(US)
Inventor: Sohngen, Gary W.
2101 Paseo Del Mar
San Pedro California 90732(US)

(74) Representative: Lehmann, Klaus, Dipl.-Ing. et
al
Schroeter, Fleuchaus, Lehmann, Wehser,
Holzer & Gallo Patentanwälte
Lipowskystrasse 10
D-8000 München 70(DE)

(54) Intramedullary rod for femur stabilization.

(57) A bowed intramedullary rod in the form of a
partially groove (40), fluted (24, 26) cylinder having a
passage (30) therethrough, one end (32) of the pas-
sage (30) being threaded to permit attachment of a
driver at one end (20), referred to for convenience as
the proximal end (20), and slightly tapered (28) to a
bullet-like point at the other or distal end (22) thereof
is described.

FIG. 2

# INTRAMEDULLARY ROD FOR FEMUR STABILIZATION

### Field of the Invention

This invention relates to surgical implements, and more particularly, this invention relates to orthopedic surgery and to appliances for fixing fractured bones, most specifically, the human femur.

### Background of the Invention

The human femur is the large bone which extends from the hip to the knee and which, consequently, carries the weight of the individual during standing walking, and doing many bodily activities.

When the human femur is fractured, it becomes necessary to assure that the fragments of the bone are aligned and that they are fixed in an aligned relationship during the period of healing. Sometimes, it is necessary to provide internal fixation in the form of an intramedullary rod which is inserted along substantially the length, or a greater portion of the length, of the femur inside the femur and which is held by nails, screws or other fixation devices. The femoral intramedullary rod, in various configurations, is well known in the art. The present invention constitutes and improved femoral intramedullary rod.

The techniques for implanting intramedullary rods is well known and, generally, such techniques are applicable for the use of this invention, with no more than ordinary modification to take advantage of the unique and advantageous configuration of the intramedullary rod which is disclosed in this patent. Typically, citing devices are provided and image intensifiers may be used to permit the surgeon to place fixative screws in the appropriate locations. Any conventional screw of suitable size and length may be used in connection with the present invention.

### Brief Description of the Drawings

Figure 1 is referred to as a top view of the intramedullary rod of this invention, recognizing that the rod has no particular top or bottom, the term being used merely to indicate one orientation as compared with a different orientation shown in Figure 2.

Figure 2 is referred to as a side view of the intramedullary rod of this invention, being taken at an angle of 90° with respect to the view shown in Figure 1.

Figure 3 is a cross-sectional view of the intramedullary rod taken substantially along the lines 3-3 in Figure 1.

Figure 4 is a cross-sectional view of just the distal tip of the intramedullary rod of this invention taken in the direction of the arrows as shown in 4-4 in Figure 1.

Figure 5 is an end view of the intramedullary rod taken from the right end, as shown on the drawing of Figure 1.

Figure 6 is a rough schematic depiction of the human femur showing generally the major structures thereof and including lines $L_1$ through $L_6$ as an aid in explaining the location and fixation of the intramedullary rod of this invention.

Figure 7 is a schematic view of the intramedullary rod of this invention shown, very schematically, in a human femur using one of the available proximal fixation orientations.

Figure 8 is comparable to Figure 7 and likewise depicts, in a very schematic manner, the intramedullary rod of this invention in a human femur, the screw in the proximal end being in the alternative angle and location as compared with Figure 7.

### Summary of the Invention

The present invention is an intramedullary rod for use in stabilizing a fractured human femur in surgical procedures. The rod comprises an elongate, generally cylindrical rod defined by a length, a diameter and a curved center line which coincides generally with the center of the rod. The curved center line lies substantially in a single plane in a first plane and defines a curve in a second plane at substantially right angles to the first plane. The curve generally corresponds to the natural curvature of the human femur which is to be stabilized. The rod is preferably unitarily formed of a single piece of titanium, and is constructed and formed to prevent rotation thereof when in use and to minimize the bulk, mass and cross-section thereof. The rod defines, adjacent the proximal end, a fastener passage therethrough. The fastener passage is defined by a diameter and a center line, said fastener passage center line lying at an angle to the curved center line of the rod which generally coincides with the angle defined by the curved center line of the rod of the human femur and a line extending through the centerline of the neck to the center of the head of the human femur for receiving a surgical fastener to fix the proximal end of the intramedullary rod in the region of the human femur generally defined by the intersection of the neck with the great trochanter and the small trochanter of the human femur. The rod has at least one fastener passage adjacent the distal end of the intramedullary rod for receiving a surgical fastener to fix the distal end of the intramedullary rod proximate the lower extremity of the human femur, generally between the medial condyle and lateral condyle. The intramedullary rod extends, when in use, through substantially the length of the human femur approximately along the centerline thereof.

The intramedullary rod defines, adjacent the proximal end, a fastener passage therethrough, said fastener passage being defined by a diameter and a center line, said fastener passage center line lying at an angle to the center line of the rod which generally coincides with the angle defined by the center line of the rod of the human femur and a line extending through the between the extremities of the great trochanter and the small trochanter of the human femur.

Typically the intramedullary rod defines a cannula proximate each end and, adjacent the proximal end a first fastener passage therethrough, said first fastener passage being defined by a diameter and a center line, said fastener passage center line lying at an angle to the curved center line of the rod which generally coincides with the angle defined by the center line of the rod of the human femur and a line extending through the between the extremities of the great trochanter and the small trochanter of the human femur, and also defines, adjacent the proximal end, a second fastener passage therethrough, said second fastener passage being defined by a diameter and a center line, said second fastener passage center line lying at an angle to the center line of the rod which generally coincides with the angle defined by the center line of the rod of the human femur and a line extending through the centerline of the neck to the center of the head of the human femur.

The intramedullary rod defines a groove extending from a point centrally from and proximate to the fastener passages adjacent the proximal end of the rod to a point distally from the fastener passages adjacent the distal end of the rod. The rod typically defines a central passage interiorly thereof from end to end coinciding with the curved center line of the rod, said central passage being defined proximate the ends by generally cylindrical passages formed coincident with the curved center line of the rod and centrally by a groove extending from a point centrally from and proximate to the fastener passages adjacent the proximal end of the rod to a point distally from the fastener passages adjacent the distal end of the rod. The rod preferably further defines flutes on opposite sides thereof extending from proximate one end thereof to proximate the other end thereof.

The first and second fastener passages adjacent the proximal end of the rod, in the exemplary embodiment, intersect each other at approximately 90 degrees to each other in a first plane and at approximately 30 degrees in a second plane substantially at right angles to the first plane.

The central passage along the length of the rod permits the rod to follow a guide wire which is inserted in the femur preliminary to installing the rod. The external surface of the rod has the groove described above and flutes. The groove intersects and partially coincides with the central passage. The surface defines at least one flute and, as depicted, preferable defines at least two flutes formed therein extending from substantially end to end thereof. The combination of annular ends, a groove as described and the flutes reduce the mass and cross-section of the rod along substantially the entire length thereof. The rod is, in the preferred embodiment, made of a single piece of titanium but may be fabricated in sections which are welded or fastened together and/or formed of another bio-compatible material of adequate strength and stiffness. The combination of material and design provides the maximum strength and rigidity along with minimum mass and cross-section and also prevents rotation of the rod, a combination not heretofore attainable.

Description of the Preferred Embodiment

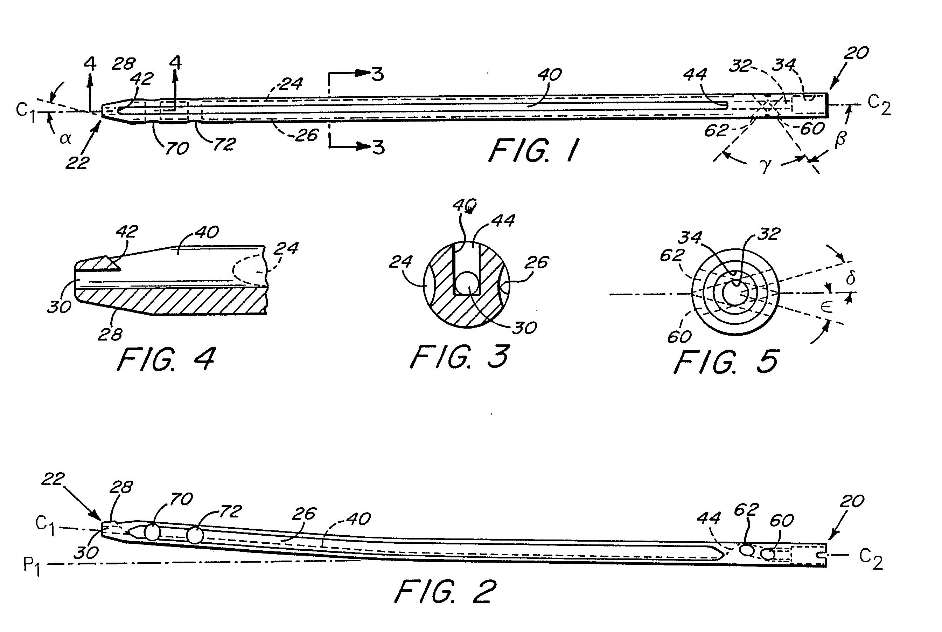

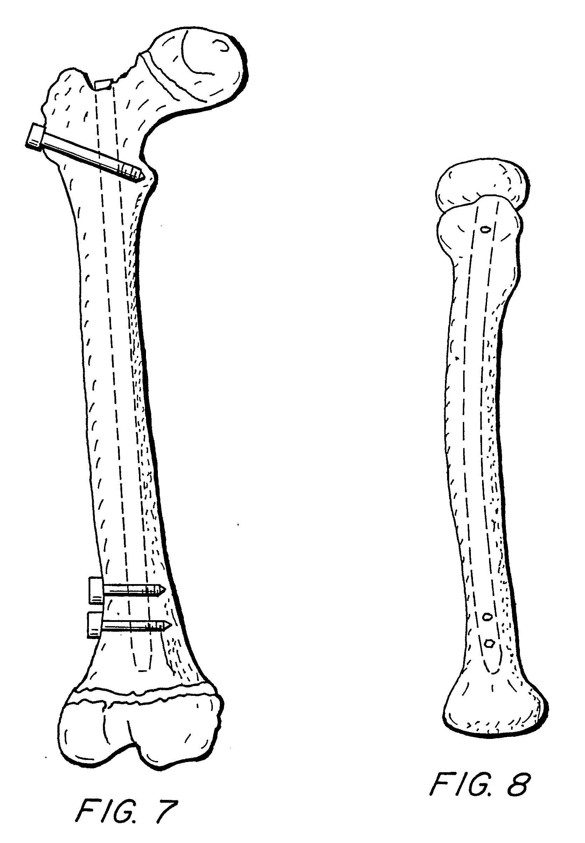

The intramedullary rod of this invention, in its preferred embodiment, is shown in Figures 1 through 5 and is depicted, in a very schematic manner, in use in Figures 7 and 8. Reference is made first to Figures 1 through 5 which show the structural features of the intramedullary rod.

The intramedullary rod comprises an elongate, generally cylindrical rod defined by a length between the proximal end 20 and the distal end 22, a diameter and a curved center line $C_1 - C_2$ which coincides generally with the center of the intramedullary rod. Figure 1 depicts what is referred to strictly for convenience as a "top" view of the intramedullary rod and Figure 2 depicts a "side" view of the same, the terms "top" and "side" being used merely to indicate first and second views taken at approximately right angles one to another. The curved center line $C_1-C_2$ lies substantially in a single plane, which is perpendicular to the sheet on which the drawings appear, when viewed in a first direction, i.e. the "top" view of Figure 1. The

curved center line $C_1$-$C_2$ defines a curve when viewed in a second direction, i.e. the "side" view of Figure 2, which is substantially right angles to the plane defined respecting the first direction of view. Stated differently, the intramedullary rod is bent substantially in only one plane. A small amount of bending in more than one plane may not be detrimental but is not necessary.

The curve in which the intramedullary rod is bent as viewed in the second direction may be defined in reference to a plane perpendicular to the plane of the sheet on which the drawing appears and defined in Figure 2 by the line $P_1$-$C_2$. The intramedullary rod is bent, in the preferred form, along a circular arc having a radius of curvature of from about 11 to 15 feet, usually approximately 13 feet.

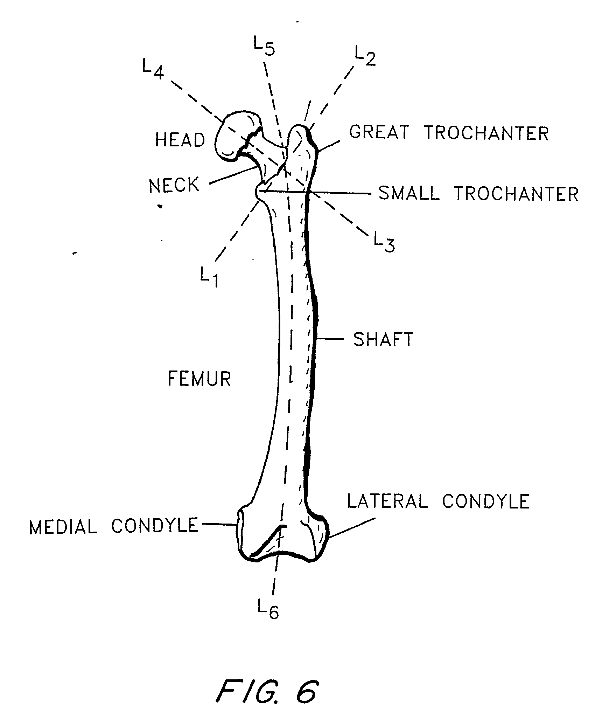

Referring briefly to Figures 5 - 7, which depict a typical human femur bone, it will be noted that the rod of the femur is not straight but, rather, is curved. The curve of the intramedullary rod of this invention generally corresponds to the natural curvature of the human femur which is to be stabilized. The curve of the intramedullary rod need not be a circular arc, but it is easier to manufacture the rod using a circular arc than other arcs which may be used. Likewise, the radius of the arc is not critical so long as the curve of the intramedullary rod generally corresponds to the natural curvature of the human femur which is to be stabilized.

Referring now to Figures 1,3 and 4 in particular, the external configuration of the intramedullary rod is defined by the length, diameter and curvature as described, and also by flutes 24 and 26 which are milled from the distal end 22 to proximate the proximal end 20 in opposing walls of the intramedullary rod, by central passage way which includes a groove which will be described in detail, and by a "bullet" shaped distal end which is tapered in a generally frustoconical configuration, the conical walls being indicated at 28. While the angle Alpha defined by the curved center line $C_1$-$C_2$ of the intramedullary rod and the walls 28 of the frustoconical "bullet" shaped distal end is not at all critical, it is generally from about 5 to 20 degrees, typically about 10 degrees. The outer configuration of the intramedullary rod is also defined by elongate flutes in a generally cylindrical wall. The outer wall of the intramedullary rod may be described as generally cylindrical, in that the overall configuration resembles a right cylinder, with fluted wall surfaces, i.e. at least one flute and preferably two flutes, or more in larger rods, running lengthwise in the cylindrical walls.

A central passageway 30 extends from the distal end 22 to the proximal end 20 of the intramedullary rod. This central passageway is defined at the distal end 22 by a round passage 30, best shown in Figure 3, drilled along the length of the rod and by a groove 40 along a major portion of the length of the intramedullary rod, the bottom of the groove 40 coinciding with the passage 30, thus defining in the center of the rod the same space. Proximate the proximal end of the intramedullary rod, the drilled passage 30 forms a threaded passage 32 and, at the proximal end of the rod, an enlarged guide opening 34. The groove 40 may, conveniently, be formed simply by sawing the groove in the wall, leaving arcuate terminal portions 42 and 44 at the respective ends, though the manner of formation is immaterial. As will be seen from Figures 1 and 2, the central passageway coincides generally with the curved center line $C_1$-$C_2$, $P_1$-$C_2$.

The central passageway defined in the intramedullary rod may, thus, be described as a central passage interior to and generally centrally located in the intramedullary rod extending from end to end coinciding with the curved center line of the intramedullary rod configuration of the intramedullary rod. The central passage is defined proximate the ends by generally cylindrical passages formed coincident with the curved center line of the intramedullary rod and centrally by a groove extending inwardly from the wall of the intramedullary rod.

Referring now briefly to Figure 6, it will be noted that there are two bony masses formed at the upper end of the femur. One mass may be described as lying along a plane defined by the line $L_1$-$L_2$ which extends from the small trochanter to the great trochanter. One mass may be described by reference to the line $L_3$-$L_4$ which extend from the central area of the head, through the axis of the neck to the area where the femur rod intersects the great trochanter. The line $L_3$-$L_4$ is defined quite precisely by the axis of the neck, whereas the line $L_1$-$L_2$ may more precisely be described as a pencil of lines which extend generally from at or adjacent to the maximum enlargement of the small and great trochanters, respectively; however, since, for present purposes, any line of this pencil of lines is functionally equivalent to all other such lines, reference hereinafter will be made to the line $L_1$-$L_2$ with the understanding that it may be any of the defined pencil of lines. Before leaving Figure 6, it will be noticed that the lines $L_1$-$L_2$ and $L_3$-$L_4$ approximately intersect a line $L_5$-$L_6$ which extends from end to end of the femur generally concentrically to the femur rod and in the central area of each of the end bony masses formed where the neck joins the small trochanter and great trochanter, at the top of the femur, and by the medial condyle and lateral condyle, at the lower end of the femur.

Referring again to Figures 1, 2 and 5, the rod of the intramedullary rod defines, adjacent the proximal end, at least one and preferably two fastener passages 60 and 62. The first fastener passage 60 is defined by a diameter and a center line, the center line thereof lying at an angle to the center line of the intramedullary rod. The angle Beta defined by the center line $C_1$-$C_2$ of the intramedullary rod axis and the axis of the first fastener passage is about 40 to 50 degrees, 45° typically, and generally coincides with the angle defined by the center line of the rod of the human femur and a line $L_3$-$L_4$ extending through the centerline of the neck to the center of the head of the human femur. In its preferred form, the intramedullary rod also has formed therein, adjacent the proximal end thereof, a second fastener passage. The second fastener passage 62 is defined by a diameter and a .center line, said fastener passage center also line lying at an angle Beta of about 45 ± 5 degrees to the center line of the intramedullary rod, and generally at right angles to the first passage center line. The second passage center line generally coincides with the angle defined by the center line $L_5$-$L_6$ of the rod of the human femur and a line $L_1$-$L_2$ extending generally through the extremities of the great trochanter and the small trochanter of the human femur.

The first and second fastener passages, which are adjacent the proximal end of the intramedullary rod, preferably intersect each other at approximately right angles, as viewed from the "top" as shown in Figure 1 but are offset from each other circumferentially on the intramedullary rod. As best shown in Figure 5, the offset angle Delta is about 20 to 40 degrees, typically approximately 30 degrees.

Stated differently, the preferred embodiment of the invention may be a intramedullary rod as described in which two intersecting fastener passages are drilled, or otherwise formed. If the intramedullary rod were transparent, and viewed through the wall of the intramedullary rod from a direction generally perpendicular to the axes of the fastener passages, the passages intersect each other at about 90 degrees, more or less, as described. This description is provided to help visualize what is clearly shown in the drawings, and it will be understood that one cannot view the fastener passages in this manner because the intramedullary rod is not transparent. Furthermore, it is not possible to view both of the fastener passages at the same time at precisely right angles because they do not lie in the same plane. However, the offset angle Delta is not so large as to render the visualization example invalid. As viewed from the proximal end of the intramedullary rod, one would see (if the rod were transparent) the two fastener passageways intersecting each other but offset around the circumference by about 30 degrees rather than lying in the same plane.

The first and second fastener passages are typically chamfered and formed in accordance with good manufacturing practices, as are all other structures, and are constructed to receive a surgical fastener, such as any of several surgical cancellous or cortical screws, to fix the proximal end of the intramedullary rod in the bone mass in the region of the human femur generally defined by the intersection of the neck with the great trochanter and the small trochanter of the human femur.

At least one fastener passage, and preferably two, indicated at 70 and 72, are drilled or otherwise formed through intramedullary rod, generally perpendicular to the center line $C_1$-$C_2$ of the intramedullary rod, adjacent the distal end of the intramedullary rod for receiving surgical fastener screws to fix the distal end of the intramedullary rod proximate the lower extremity of the human femur on the bony mass in the general region between the medial condyle and lateral condyle thereof.

Referring now to Figures 7 and 8, it will be see that, in use, the intramedullary rod extends through substantially the length of the human femur approximately along the center line $L_5$-$L_6$ thereof.

The intramedullary rod of this invention is preferably manufactured of titanium surgical implant alloy (6AL4VELI), ASTM Standard F-136-84. Other bio-compatible alloys may be used, such as various stainless steels, e.g. 316-L stainless steel; however, the weight-to-strength ratio of titanium makes it vastly superior to other materials. The intramedullary rod is typically produced in several sequential sizes. For example, diameters of 13 mm, 14 mm and 15 mm, and lengths of 40 cm, 42 cm and 44 cm are relatively standard sizes. Other sizes are in less demand but are manufactured as needed.

The physical configuration of the intramedullary rod has been described in considerable detail with respect to the Figures. Generally, however, the intramedullary rod may be described as a partially groove, fluted cylinder having a passage therethrough. Preferably one end of the passage is threaded to permit attachment of a driver at one end, referred to for convenience as the proximal end, and the rod is slightly tapered to a bullet-like point at the other or distal end thereof. The rod is bowed. Typically, the radius of the bow is about 13 feet, but as long as there is a bow to approximate the natural anterior curve of the human femur, any such suitable radius of curvature may be used. This is one of the important features of the invention. The bowing of the rod, coupled with the groove and fluted configuration, permits the intramedullary rod of this invention to be inserted into the femur with minimum displacement and removal of bone marrow, and the use of titanium, provides strength, rigidity and prevents rotation when in use, and, in addition, allows the marrow and tissue to grow more normally, leaving more space for tissue growth, and permits more rapid healing than is the case with the prior art intramedullary rods. The rod is preferably produced from a solid bar of surgical implant-grade titanium alloy which has been machined so as to produce a longitudinal external flutes and a deep lengthwise groove, with access for a guide wire and apertures or passages for proximal, medial and distal perpendicular holes to accommodate anchoring screws, all as described. The anchoring screws are provided in two types, cancellous and cortical, and are made of the same titanium alloy as the rod. Screws are available in several sizes such as, for example, 6.5mm in diameter X 50mm, 60mm, 70mm or 80mm in length.

The intramedullary rod of this invention is configured and dimensioned to accept a guide wire made, typically, from 316-L stainless steel, the guide wire having a diameter of 1,8 inch and, typically, a length of about three feet.

The intramedullary rod of this invention is intended for use in the femur and is "universal" in design in that there is no "right" or "left" devised configuration.

It will be understood that minor variations in dimensions, angles of curvature, radius of curvature, etc., as well as in materials, as long as suitable biologically compatible materials are used, may be used without departing from the spirit and scope of the invention.

Industrial Application

This invention finds industrial application in human and veterinary surgery, but principally in orthopedic surgery on the human being.

**Claims**

1. An intramedullary rod for use in stabilizing a fractured human femur in surgical procedures comprising: an elongate, generally cylindrical rod defined by a length, a diameter and a curved center line which coincides generally with the center of the rod, the curved center line lying substantially in a single plane in a first plane and defining a curve in a second plane at substantially right angles to the first plane, the curve generally corresponding to the natural curvature of the human femur which is to be stabilized, the rod defining a passage from end to end thereof and a groove encompassing the passage and extending to the side of the rod along the rod from a point proximate the proximal end to a point proximate the distal end of the rod, the rod defining adjacent the proximal end a fastener passage therethrough at an angle to the center line of the rod corresponding to a line extending generally through the centerline of the neck of the femur to proximate the center of the head of the human femur for receiving a surgical fastener to fix the proximal end of the intramedullary rod in the region of the human femur generally defined by the intersection of the neck with the center line of the femur, the rod further defining at least one fastener passage adjacent the distal end of the intramedullary rod for receiving a surgical fastener to fix the distal end of the intramedullary rod proximate the lower extremity of the human femur generally between the medial condyle and lateral condyle thereof, the intramedullary rod extending, when in use, through substantially the length of the human femur approximately along the centerline thereof.

2. An intramedullary rod for use in stabilizing a fractured human femur in surgical procedures comprising: an elongate, generally cylindrical rod defined by a length, a diameter and a curved center line which coincides generally with the center of the rod, the curved center line lying substantially in a single plane in a first plane and defining a curve in a second plane at substantially right angles to the first plane, the curve generally corresponding to the natural curvature of the human femur which is to be stabilized, the rod defining a passage from end to end thereof and a groove encompassing the passage and extending to the side of the rod along the rod from a point proximate the proximal end to a point proximate the distal end of the rod, the rod defining adjacent the proximal end a fastener passage therethrough at an angle to the center line of the rod corresponding to a line extending through a trochanter center line extending from proximate the center of the small trochanter to proximate the center of the great trochanter of the human femur for receiving a surgical fastener to fix the proximal end of the intramedullary rod in the region of the human femur generally defined by the great trochanter and the small trochanter of the human femur, the rod further defining at least one fastener passage adjacent the distal end of the intramedullary rod for receiving a surgical fastener to fix the distal end of the intramedullary rod proximate the lower extremity of the human femur generally between the medial condyle and lateral condyle thereof, the intramedullary rod extending, when in use, through substantially the length of the human femur approximately along the centerline thereof.

3. An intramedullary rod for use in stabilizing a fractured human femur in surgical procedures comprising: an elongate, generally cylindrical rod (10) defined by a length, a diameter and a curved center line $(C_1-C_2, P_1-C_2)$ which coincides generally with the center of the rod (10), the curved center line lying substantially in a single plane in a first plane and defining a curve in a second plane at substantially right angles to the first plane, the curve generally corresponding to the natural curvature of the human femur which is to be stabilized, the rod defining a passage (30) from end to end thereof and a groove (40) encompassing the passage and extending to the side of the rod along the rod from a point (44) proximate the proximal end to a point (42) proximate the distal end of the rod, the rod defining adjacent the proximal end a first fastener passage (60) therethrough at an angle to the center line of the rod corresponding to a line extending generally through the centerline of the neck of the femur to proximate the center of the head of the human femur for receiving a surgical fastener to fix the proximal end of the intramedullary rod in the region of the human femur generally defined by the intersection of the neck with the center line of the femur and a second fastener passage (62) therethrough at an angle to the center line of the rod corresponding to a line extending through a trochanter center line extending from proximate the center of the small trochanter to proximate the center of the great trochanter of the human femur for receiving a surgical fastener to fix the proximal end of the intramedullary rod in the region of the human femur generally defined by the great trochanter and the small trochanter of the human femur, the rod further defining at least one fastener passage (70, 72) adjacent the distal end of the intramedullary rod for receiving a surgical fastener to fix the distal end of the intramedullary rod proximate the lower extremity of the human femur generally between the medial condyle and lateral condyle thereof, the intramedullary rod extending, when in use, through substantially the length of the human femur approximately along the centerline thereof.

4. The intramedullary rod of Claim 3 comprising a single internal titanium rod wherein the first and second fastener passages adjacent the proximal end of the rod intersect each other.

5. The intramedullary rod of Claim 3 wherein the first and second fastener passages adjacent the proximal end of the rod intersect each other at an angle Beta of approximately 90 degrees to each other in a first plane and an angle Delta at approximately 30 degrees in a second plane substantially at right angles to the first plane.

6. The intramedullary rod of Claim 5 wherein the circumferential surface of the rod further defines at least two flutes (24, 26) therein.

7. The intramedullary rod of Claim 6 wherein the flutes (24, 26) are on opposite sides of the rod approximately equally spaced from the groove (40) therein.

8. The intramedullary rod of Claim 7 wherein in the rod defines a generally frustoconical distal end.

9. The intramedullary rod of Claim 3 wherein the circumferential surface of the rod further defines at least two flutes (24, 26) therein.

10. The intramedullary rod of Claim 9 wherein the flutes (24, 26) are on opposite sides of the rod approximately equally spaced from the groove (40) therein.

11. The intramedullary rod of Claim 10 wherein in the rod defines a generally frustoconical distal end.

12. The intramedullary rod of Claim 3 wherein in the rod defines a generally frustoconical distal end.

13. An intramedullary rod for use in stabilizing a fractured human femur in surgical procedures comprising: an elongate, generally cylindrical unitarily formed titanium rod defined by a length, a diameter and a curved center line which coincides generally with the center of the rod, the curved center line lying substantially in a single plane in a first plane and defining a curve in a second plane at substantially right angles to the first plane, the curve generally corresponding to the natural curvature of the human femur which is to be stabilized, the rod defining a passage from end to end thereof and a groove encompassing the passage and extending to the side of the rod along the rod from a point proximate the proximal end to a point proximate the distal end of the rod, the rod defining adjacent the proximal end a fastener passage therethrough at an angle to the center line of the rod corresponding to a line extending generally through the centerline of the neck of the femur to proximate the center of the head of the human femur for receiving a surgical fastener to fix the proximal end of the intramedullary rod in the region of the human femur generally defined by the intersection of the neck with the center line of the femur, the rod further defining at least one fastener passage adjacent the distal end of the intramedullary rod for receiving a surgical fastener to fix the distal end of the intramedullary rod proximate the lower extremity of the human femur generally between the medial condyle and lateral condyle thereof, the intramedullary rod extending, when in use, through substantially the length of the human femur approximately along the centerline thereof.

14. An intramedullary rod for use in stabilizing a fractured human femur in surgical procedures comprising: an elongate, generally cylindrical titanium rod defined by a length, a diameter and a curved center line which coincides generally with the center of the rod, the curved center line lying substantially in a single plane in a first plane and defining a curve in a second plane at substantially right angles to the first plane, the curve generally corresponding to the natural curvature of the human femur which is to be stabilized, the rod defining a passage from end to end thereof and a groove encompassing the passage and extending to the side of the rod along the rod from a point proximate the proximal end to a point proximate the distal end of the rod, the rod defining adjacent the proximal end a fastener passage therethrough at an angle to the center line of the rod corresponding to a line extending through a trochanter center line extending from proximate the center of the small trochanter to proximate the center of the great trochanter of the human femur for receiving a surgical fastener to fix the proximal end of the intramedullary rod in the region of the human femur generally defined by the great trochanter and the small trochanter of the human femur, the rod further defining at least one fastener passage adjacent the distal end of the intramedullary rod for receiving a surgical fastener to fix the distal end of the intramedullary rod proximate the lower extremity of the human femur generally between the medial condyle and lateral condyle thereof, the intramedullary rod extending, when in use, through substantially the length of the human femur approximately along the centerline thereof.

15. An intramedullary rod for use in stabilizing a fractured human femur in surgical procedures comprising: an elongate, generally titanium cylindrical rod (10) defined by a length, a diameter and a curved center line $(C_1 \text{-} C_2, P_1 \text{-} C_2)$ which coincides generally with the center of the rod (10), the curved center line lying substantially in a single plane in a first plane and defining a curve in a second plane at substantially right angles to the first plane, the curve generally corresponding to the natural curvature of the human femur which is to be stabilized, the rod defining a passage (30) from end to end thereof and a groove (40) encompassing the passage and extending to the side of the rod along the rod from a point (44) proximate the proximal end to a point (42) proximate the distal end of the rod, the rod defining adjacent the proximal end a first fastener passage (60) therethrough at an angle to the center line of the rod corresponding to a line extending generally through the centerline of the neck of the femur to proximate the center of the head of the human femur for receiving a surgical fastener to fix the proximal end of the intramedullary rod in the region of the human femur generally defined by the intersection of the neck with the center line of the femur and a second fastener passage (62) therethrough at an angle to the center line of the rod corresponding to a line extending through a trochanter center line extending from proximate the center of the small trochanter to proximate the center of the great trochanter of the human femur for receiving a surgical fastener to fix the proximal end of the intramedullary rod in the region of the human femur generally defined by the great trochanter and the small trochanter of the human femur, the rod further defining at least one fastener passage (70, 72) adjacent the distal end of the intramedullary rod for receiving a surgical fastener to fix the distal end of the intramedullary rod proximate the lower extremity of the human femur generally between the medial condyle and lateral condyle thereof, the intramedullary rod extending, when in use, through substantially the length of the human femur approximately along the centerline thereof.

16. The intramedullary rod of Claim 15 wherein the first and second fastener passages adjacent the proximal end of the rod intersect each other at an angle Beta of approximately 90 degrees to each other in a first plane and an angle Delta at approximately 30 degrees in a second plane substantially at right angles to the first plane.

17. The intramedullary rod of Claim 15 wherein the rod defines flutes (24, 26) on opposite sides of the rod approximately equally spaced from the groove (40) therein.

18. The intramedullary rod of Claim 17 wherein in the rod defines a generally frustoconical distal end.

FIG. 1

FIG. 4

FIG. 3

FIG. 5

FIG. 2

EP 0 355 411 A1

FIG. 6

FIG. 7

FIG. 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 475 545  (ENDER) <br> * Column 1, lines 19-37; column 2, lines 41-44; column 3, line 9 - column 4, line 67; figures * | 1-4,13-15 | A 61 B  17/58 |
| Y | | 9,12,17 | |
| A | | 5,8 | |
| Y | DE-A-3 324 103  (ROTTHÄUSER-REIMER) <br> * Page 15, lines 9-11; figures 1-5 * | 9,17 | |
| A | | 6 | |
| Y | US-A-4 622 959  (MARCUS) <br> * Column 4, line 35 - column 6, line 17; column 8, lines 44,45; figures 1-4 * | 12 | |
| A | | 1-6,8,9,11,13-18 | |
| A,P | GB-A-2 209 947  (HALDER) | | |
| A | EP-A-0 273 872  (CREMASCOLI) | | TECHNICAL FIELDS SEARCHED (Int. Cl.5) |
| A | DE-A-3 537 318  (VEB K.M.L.L.) | | A 61 B |

The present search report has been drawn up for all claims

| Place of search | Date of completion of the search | Examiner |
|---|---|---|
| THE HAGUE | 20-11-1989 | KLEIN C. |